# EUROPÄISCHE PATENTSCHRIFT

(11) **EP 0 552 130 B1**
(45) Veröffentlichungstag und Bekanntmachung des Hinweises auf die Patenterteilung: **20.03.1996**
(21) Anmeldenummer: 93810008.8
(22) Anmeldetag: 11.01.1993
(51) Int. Cl.: C07C 205/05, C07C 201/08, B01J 29/70

(54) **Verfahren zur Herstellung von Nitrobenzol**
Process for the preparation of nitrobenzene
Procédé de préparation de nitrobenzène

(30) Priorität: 15.01.1992 CH 101/92
(43) Veröffentlichungstag der Anmeldung: 21.07.1993
(73) Patentinhaber: CU CHEMIE UETIKON AG, CH-8707 Uetikon am See (CH)
(72) Erfinder: Kouwenhoven, Herman W., CH-8704 Herrliberg (CH); Bertea, Leopoldo, CH-8052 Zürich (CH); Prins, Roel, CH-8032 Zürich (CH)
(74) Vertreter: Werffeli, Heinz R., Dipl.-Ing.ETH.

(56) Entgegenhaltungen:
- EP-A- 0 053 031
- CHEMICAL ABSTRACTS, vol. 110, 1989, Columbus, Ohio, US; Zusammenfassung Nr. 57294t, M. FURUYA et al: "Preparation of aromatic mononitro compounds by catalytic nitration with nitric acid", Seite 651, Spalte 1; & JP-A-63 225 339
- Atlas of Zeolitic Structure, W.M. Meier und D.H. Olson, Butterworth-Heinemann, 3. Aufl., Seite 106

## Beschreibung

Gegenstand der Erfindung ist ein Verfahren zur Herstellung von Nitrobenzol durch Nitrierung von Benzol in der Gasphase mit wässriger Salpetersäure mit einem Salpetersäure Gehalt von 15 bis 95 Gew%, vorzugsweise von mehr als 50 Gew% in Gegenwart von auf Klinoptilolit basierenden Katalysatoren.

Verfahren zur Gasphasennitrierung von Aromaten, in denen NO₂ oder andere Stickoxide als Nitriermittel angewandt werden, sind schon mehrfach in der einschlägigen Literatur beschrieben worden. Eine Übersicht in US-Pat. 4.754.083 zeigt, dass die Anwendung einer grossen Anzahl von unterschiedlichen Materialien als Katalysatoren für die Gasphasennitrierung von Aromaten mit Stickoxyden beansprucht wurde. Zeolithe und andere kristalline und amorphe Aluminosilikate werden in dieser Übersicht besonders häufig als Katalysatoren erwähnt. Die negative elektrische Ladung des Zeolith-Gitters, verursacht durch die Substitution von Silizium- durch Aluminium Atome, wird durch zusätzliche Metallionen ausgeglichen. Diese ausgleichenden Metallionen können häufig leicht mit Ionenaustauschverfahren durch andere Ionen ersetzt werden. In vielen Anwendungen der Katalyse werden saure, meistens als H-Zeolithe bezeichnete Zeolithe benützt. H-Zeolithe werden entweder direkt durch Ionenaustausch mit Säuren oder indirekt durch eine Kalzinierung von einem Ammonium-Zeolithen hergestellt. Ein Ammonium-Zeolith wird als Zwischenstufe durch Ionenaustausch von einem Zeolithen mit einer Lösung eines Ammoniumsalzes hergestellt.

In US-Pat. 4.754.083 werden Molekularsiebe, Montmorillonit und mittels sog. Pfeilern ausgeweiteter Bentonit im allgemeinen als Katalysatoren für die Gasphasennitrierung von substituierten Aromaten, welche eine meta-dirigierende Gruppe wie -NO₂ enthalten, beansprucht. Eine grosse Anzahl von unterschiedlichen Zeolithen, unter anderen Zeolon 900H, H-Y, H-Beta, H-ZSM5, Na-X, H-Ferrierit und H-Erionit werden im US-Pat. 4.754.083 als Katalysatoren für die Gasphasennitrierung von Nitrobenzol angewandt. In der Beschreibung im US-Pat. 4.754.083 der durchgeführten Experimente wird die Anwendung von zwei verschiedenen Reaktoren erwähnt. Ergebnisse über Umsatz, gemessen in beiden Reaktortypen nach ein bis zwei Stunden Reaktionsdauer, werden beschrieben. Diese Daten sind somit nicht ausreichend, um Aussagen über die Langzeitstabilität der Katalysator-Leistung machen zu können. In der EP-A 343.048 wird ein Gasphasenverfahren zur Herstellung von Nitrobenzol mit Salpetersäure vorgeschlagen, in der Katalysatoren basierend auf sauren Schichtsilikaten oder sauren, oxidischen Materialien angewandt werden, die Oxide aus der Gruppe IVA des periodischen Systems zusammen mit Wolframtrioxid, Molybdäntrioxid oder Zinkoxid umfassen. Stabilität, Aktivität und Selektivität der in der EP-A 343.048 erwähnten bevorzugten Katalysatoren sollen höher sein als bei den früher beschriebenen Katalysatoren.

Die Anwendung von heterogenen Katalysatoren, von denen einige auf Zeolon 900H basieren, in der Gasphasennitrierung von namentlich Chlorbenzol und Benzol mittels NO₂, N₂O₄, oder N₂O₃, vorzugsweise mit NO₂, wird in einer Reihe von Patenten, die u.a. EP-A 053.031; EP-A 053.053; EP-A 078.247; EP-A 092.372 und US-Pat. 4.107.220 umfasst, beschrieben. In EP-A 053.031 wird angegeben dass nahezu jeder damals bekannte Zeolith, unabhängig von Zusammensetzung, Textur oder chemische Stabilität, bei der Gasphasennitrierung von nahezu allen monoaromatischen Kohlenwasserstoffen, aromatischen Ethern und Haloaromaten, namentlich mit nahezu allen bekannten Stickstoffoxyden als Nitriermitteln mit Erfolg als Katalysator eingesetzt werden kann. Ausser einer Vorbehandlung bei Reaktionsbedingungen mit NO₂, dem bevorzugten Nitriermittel, wird keine weitere, spezifische Vorbehandlung als wichtig für die Katalysator-Leistung angesehen. In EP-A 053.031 wird ausserdem gezeigt, dass amorphes Silica-Alumina eine ähnliche, oder etwas bessere, Katalysator-Leistung als Zeolon-900H hat.Ausser Zeolon 900H wurden keine weitere Zeolithe überprüft. In den späteren Patenten der obengenannten Reihe werden Massnahmen beschrieben, die zu einer verbesserten Katalysator-Leistung führen. Die besten Katalysatoren enthalten eine beträchtliche Menge SO₃, zB Zeolon 900H und SiO₂ bis zu 18 Gew%, und wurden durch eine wiederholte Behandlung mit einem Gemisch von SO₂ und NO₂ dargestellt. Ohne Vorbehandlung mit SO₂/NO₂ hat Zeolon 900H eine mittelmässige Katalysator-Leistung. Die katalytische Versuche, die in dieser obengenannten Reihe von Patenten beschrieben werden, wurden nach 6 - 7 Stunden Reaktionsdauer beendet. Die Daten sind somit nicht ausreichend, um Aussagen über die Langzeitstabilität der Katalysator-Leistung machen zu können.

In der DE-OS 2,826,433 und im US-Pat 4.418.230, beide von J.M.Bakke und J.Liaskar, wird H-Mordenit als Katalysator für die Gasphasennitrierung von Toluol mit Salpetersäure angewandt. Die Versuche wurden bei Normaldruck und 473K und einem Molverhältnis von Aromat und Salpetersäure von etwa 1,4 durchgeführt Daten über die Stabilität der Katalysator-Leistung werden nicht gegeben. Auch in diesen Patenten wird keine weitere Vorbehandlung des Zeolithen als wichtig für die Katalysator-Leistung genannt. Die Leistung von dem auf Mordenit basierenden Katalysator ist niedrig im Vergleich mit der eines auf Montmorillonit basierenden Katalysators, und die meisten Daten beziehen sich auf Katalysatoren auf Basis Montmorillonit. Auf H-Mordenit basierende Katalysatoren, die in den oben erwähnten Patenten genannt werden, sind Handelsprodukte und wurden durch die Norton Cy als Zeolon-xxxH (zB Zeolon 200H oder Zeolon 900H) vermarktet.

Unser EP-A-0 551 052 befasst sich mit der Anwendung von auf Mordenit basierenden Katalysatoren, die in der Gasphasennitrierung von Aromaten angewendet werden und mit den Effekten von unterschiedlichen Methoden zur Aktivierung des Zeolithen. In weiteren Studien mit anderen Zeolithen haben wir überraschend gefunden, dass basierend auf Klinoptilolit hervorragende Katalysatoren für die Nitrierung von Aromaten hergestellt werden können.

Klinoptilolit ist ein Molekularsieb, das als Mineral in der Erdkruste vorkommt. Die chemische Zusammensetzung von den unterschiedlichen Klinoptiloliten ist verschieden und ist vom Fundort abhängig. Die Struktur von Klinoptilolit wird beschrieben im "Atlas of Zeolitic Structure Types" von W.M. Meier und D.H. Olson, zweite Ausgabe, im Auftrag von der "International Zeolite Association" in 1987 vom Verlag Butterworth herausgegeben. Weitere Daten über die Struktur und Eigenschaften von Klinoptiloliten findet man z.B. in einem Artikel von M.W.Ackley und R.T.Yang im AIChE Journal 37, (1991), 1645.

Die Nitrierung von Aromaten gemäss der vorliegenden Erfindung wird mit Katalysatoren basierend auf Klinoptilolit durchgeführt.Die Katalysatoren können mittels Ionenaustauschverfahren und Kalzinierungen aus Klinoptilolit hergestellt werden. Im Folge dieses Herstellungsverfahrens werden erstens die austauschbaren Metallionen teilweise oder ganz entfernt und ersetzt durch Protonen, und zweitens können Aluminiumatome aus deren herkömmlichen Gitterstellen, in der Fachliteratur mit T-Sites angedeutet, entfernt und gegebenenfalls durch Siliziumatome ersetzt werden.

Es wird vorgeschlagen die Nitrierung von Aromaten in der Gasphase bei einer Temperatur von 400 bis 523K, unter Anwendung von verdünnter oder konzentrierter, wässriger Salpetersäure mit einer Salpetersäuregehalt von 15 bis 95 Gew%, vorzugsweise von mehr als 50 Gew%, mittels auf Klinoptilolit basierenden Katalysatoren durchzuführen

Die Erfindung wird anschliessend anhand von Ausführungsbeispielen erläutert. Die Herstellung von einigen herkömmlichen Katalysatoren basierend auf Mordenit wird in Beispiel 1 beschrieben. Die Herstellung von auf Klinoptilolit basierenden Katalysatoren wird in den Beispielen 2 und 3 beschrieben. Prüfung der in Beispielen 1 bis 3 beschriebenen Katalysatoren und von Zeolon 900H wird in den Beispielen 4 bis 11, beschrie-ben.

### Herstellung der Katalysatoren

### Beispiel 1.

Bei der Herstellung der Katalysatoren wurde handelsüblicher Mordenit, hergestellt von der CU Chemie Uetikon, Typ PM1-Na, verwendet. Die analytischen Daten sind in der Tabelle 1 erwähnt. Dieses Material wurde wie folgt aktiviert: 500 g PM1-Na wurden in 5 l einer 1M Lösung von Salzsäure aufgeschlämmt und während einer Stunde unter Rühren bei 373K erhitzt. Die Aufschlämmung wurde anschliessend filtriert und mit 5 l demineralisiertem Wasser gewaschen. Der während einer Stunde bei 373K getrocknete Filterkuchen bildet Probe A. 50 g von Probe A wurden in 500 ml von einer einmolaren, wässrigen Ammoniumnitrat-Lösung aufgeschlämmt und während einer Stunde unter Rühren bei 373K geheizt. Die Aufschlämmung wurde filtriert und mit 500 ml demineralisiertem Wasser gewaschen. Das Produkt wurde noch einmal in 500 ml einer einmolaren, wässrigen Ammoniumnitrat-Lösung aufgeschlämmt und während einer Stunde unter Rühren bei 373K geheizt. Die Aufschlämmung wurde filtriert und mit 500 ml demineralisiertem Wasser gewaschen. Ein Teil des Filterkuchens wurde während 16 Stunden bei 393 K getrocknet und wahrend drei Stunden bei einer Temperatur von 773K kalziniert und bildet Probe B. Ein anderer Teil des Filterkuchens wurde in einem geschlossenen Tiegel während fünf Stunden bei 823K kalziniert und bildet Probe C. Ein dritter Teil des Filterkuchens wurde in einem geschlossenen Tiegel während fünf Stunden auf 973K kalziniert und bildet Probe D. Die analytischen Daten werden in der Tabelle 1 erwähnt.

### Beispiel 2.

Bei der Herstellung von auf Klinoptilolit basierenden Katalysatoren wurde handelsüblicher Klinoptilolit, Typ Clino MH, von der CU Chemie Uetikon verwendet. Die Klinoptilolitkristalle haben eine mittlere Grösse kleiner als 500nm und bilden grössere, harte Agglomerate. Weitere analytischen Daten werden in der Tabelle 1 erwähnt. Anmonium-Klinoptilolit wurde wie folgt hergestellt: 150 G Clino MH wurde in 500 ml einer ein-molaren Lösung von Ammoniumnitrat in Wasser aufgeschlämmt und während einer Stunde unter Rühren bei 373K erhitzt. Die Aufschlämmung wurde anschliessend filtriert und mit 1 Liter demineralisiertem Wasser gewaschen. Das Produkt wurde noch zweimahl in 500 ml einer ein-molaren Lösung von Ammoniumnitrat in Wasser aufgeschlämmt und während einer Stunde unter Rühren bei 373K erhitzt, anschliessend filtriert und mit 1 Liter demineralisiertem Wasser gewaschen. Das Produkt wurde während 85 Stunden bei 433K getrocknet und anschliessend in einer dünnen Schicht in einem offenen Tiegel in Luft wahrend drei Stunden bei 823K kalziniert. Das kalzinierte Material bildet Probe E. Die analytischen Daten sind in der Tabelle 1 erwähnt.

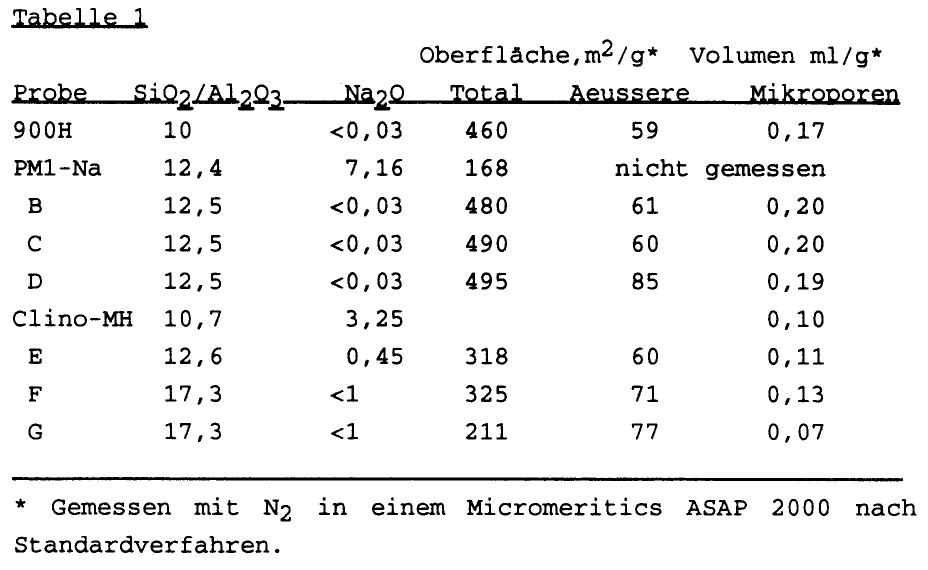

### Beispiel 3

150 G Clino MH wurde in 500 ml einer ein-molaren Lösung von Schwefelsäure aufgeschlämmt und während einer Stunde unter Rühren bei 373K erhitzt. Die Aufschlämmung wurde anschliessend filtriert und mit 1 Liter demineralisiertem Wasser gewaschen. Das Produkt wurde noch einmal in 500 ml einer einmolaren Lösung von Schwefelsäure aufgeschlämmt und während einer Stunde unter Rühren bei 373K erhitzt, anschliessend filtriert und mit 1 Liter demineralisiertem Wasser gewaschen. Das während 85 Stunden bei 433K getrocknete Produkt bildet Probe F. Ein Teil von Probe F wurde in einer dünnen Schicht in einem offenen Tiegel in Luft während drei Stunden bei 823K kalziniert und das kalzinierte Produkt bildet Probe G.
Die analytischen Daten sind in der Tabelle 1 erwähnt.

### Katalysatorprüfung

Die Gasphasennitrierung von Benzol wurde in kontinuierlichen Experimenten während mindestens 20 Stunden in einem Festbett-reaktor durchgeführt. Die folgenden Reaktionsbedingungen wurden für den Vergleich der Katalysatoren gewählt:

| | |
|---|---|
| Raumgeschwindigkeit Benzol | 1 kg/kg(Kat)Stunde |
| Raumgeschwindigkeit Salpetersäure | 0,4 |
| kg/kg(Kat)Stunde Temperatur | 443K |
| Druck | atmosph. Druck |

In den folgenden Versuchen wurde käufliche wässrige 65 Gew% Salpetersäure benützt. Das Trägergas war reiner Stickstoff. Das Volumen Verhältnis Trägergas/Benzol(Dampf) war etwa 1,3. Die Partialdrücke (in mbar) der verschiedenen Komponenten im Eduktstrom waren: Salpetersäure 138; Benzol 281; Wasser 256; Stickstoff 385.

Die Katalysatoren wurden vor Gebrauch zu einer Körnung von 0,4-0,9 mm zerkleinert. Sie wurden entweder nach einer Kalzinierung bei einer Temperatur von 773K und einer anschliessenden Aequilibrierung bei Raumtemperatur in Luft oder aber ohne vorangehende Kalzinierung im Reaktor eingesetzt und dort bei Reaktionstemperatur und Trägergasdurchfluss während mindestens zweier Stunden vorbehandelt. Daraufhin wurde der Katalysator während 30 min bei Reaktionsbedingungen mit Salpeter-säuredämpfen vorbehandelt; während dieser Behandlung wurde meistens eine Temperatursteigerung beobachtet. Die optimale Zeitdauer der Behandlung mit Salpetersäuredämpfen hängt natürlich von der eingesetzten Menge Katalysator und von den gewählten Reaktionsbedingungen ab und wird vorzugsweise betrieben, bis der Katalysator mit Salpeter-säuredämpfen gesättigt ist. Die hier angegebene Behandlungs-dauer von 30 min ist also nur als Beispiel zu betrachten. Nach Erreichen des Temperaturgleichgewichts wurde verdampftes Benzol zugemischt, auch dann wurde meistens ein exothermes Verhalten beobachtet, das durch den zeitlich bedingten Salpe-tersäure-Überschuss auf dem Katalysator verursacht wurde. Die Reaktionsprodukte wurden nach einer Einlaufperiode von einer Stunde in einer mit Methanol gefüllten Kühlfalle bei 278K gesammelt, und periodisch mittels GC in Bezug auf die organischen Anteile analysiert. Die unverbrauchte Salpetersäure wurde durch Titration ermittelt.

### Beispiele 4 bis 11

Das Verfahren zur Katalysatorprüfung nach obiger Beschreibung wurde für folgende Materialien angewandt. Beispiel 4: Zeolon 900H; Beispiel 5: Probe B; Beispiel 6: Probe C; Beispiel 7: Probe D; Beispiel 8: Clino MH; Beispiel 9: Probe E; Beispiel 10: Probe F; Beispiel 11: Probe G.

Tabelle 2 beinhaltet die Resultate, die in den unterschiedlichen, experimentellen Beispielen 4 bis 11 erhalten wurden. Die Selektivität bezüglich Benzol war in all diesen Experimenten besser als 99%. Alle Experimente wurden während mindestens 20 Stunden durchgeführt.

Die Daten in Tabelle 2 zeigen, dass bei der Gasphasen-nitrierung von Benzol die Leistung von Katalysatoren auf Basis von H-Mordenit stark durch deren Herstellungsmethode beeinflusst wird.

**Tabelle 2**

| Beispiele 4-11. | | | | | | | | |
|---|---|---|---|---|---|---|---|---|
| | Beispiele | | | | | | | |
| | 4 | 5 | 6 | 7 | 8 | 9 | 10 | 11 |
| Ausbeute Nitrobenzol bezüglich HNO3(%)* | 45/22 | 69 | 70 | 70/36 | 64/61 | 82/81 | 74/71 | 55/45 |
| Umsatz Benzol(%)* | 23/10 | 32 | 34 | 35/18 | 30/29 | 40/39 | 34/33 | 26/21 |
| Selektivität der HNO3 Benützung(%)* | 99 | 90 | 90 | 96/99 | 92 | 99+ | 93 | 89/94 |

| | | | | | | | | |
|---|---|---|---|---|---|---|---|---|
| * Falls Desaktivierung vorkam, werden zwei Werte genannt, | | | | | | | | |

wobei sich der Erste auf das Resultat nach vier Stunden und der Zweite sich auf das Resultat nach 20 Stunden bezieht.

Norton 900H zeigt im Vergleich mit den anderen auf Mordenit basierenden Katalysatoren eine niedrige Aktivität und Stabilität. Die Katalysatoren basierend auf Klinoptilolit sind aktiver als die auf Mordenit basierenden und zeigen nur eine geringe Deaktivierung während der Versuchsdauer.

## Patentansprüche

1. Verfahren zur Herstellung von Nitrobenzol, dadurch gekennzeichnet, dass man Benzol bei einer Temperatur zwischen 400 und 523 K in Gegenwart von auf Klinoptilolit basierenden Katalysatoren mittel wässriger Salpetersäure in der Gasphase nitriert.

2. Verfahren nach Anspruch 1, dadurch gekennzeichnet, dass die Klinoptilolitkristalle kleiner als 1000 nm sind.

3. Verfahren nach Anspruch 1, dadurch gekennzeichnet, dass die Nitrierung mit wässriger Salpetersäure mit einer Konzentration höher als 50 Gew% durchgeführt wird.

4. Verfahren nach den Ansprüchen 1, 2 und 3, dadurch gekennzeichnet, dass ein Teil der Alkali- und Erdalkalikationen, die im natürlichen Klinoptilolit vorhanden sind, durch Wasserstoffionen ersetzt sind.

## Claims

1. A process for the preparation of nitrobenzene, characterized by the catalytic nitration of benzene in the gasphase at a temperature between 400 and 523K with aqueous nitric acid using a catalyst based on clinoptilolite.

2. A process according to claim 1, characterized by an average clinoptilolite crystal size smaller than 1000 nm.

3. A process according to claim 1, characterized by the use of a nitrating agent consisting of aqueous nitric acid having a nitric acid content higher than 50 wt%.

4. A process according to claims 1,2 and 3 characterized by a catalyst consisting of clinoptilolite in which a fraction of the alkali- and alkaline earth-ions contained in the natural clinoptilolite are replaced by protons.

## Revendications

1. Procédé pour la fabrication de nitrobenzol, caractérisé en ce qu'on nitrifie du benzol à une température entre 400 et 523 °K en présence de catalyseurs sur base de clinoptilolite à l'aide d'acide nitrique aqueuse dans la phase gazeuse.

2. Procédé selon la revendication 1, caractérisé en ce que les cristaux de clinoptilolite sont plus petits que 1000 nm.

3. Procédé selon la revendication 1, caractérisé en ce que la nitration est effectuée avec de l'acide nitrique aqueuse avec une concentration supérieure à 50% en poids.

4. Procédé selon les revendications 1,2 et 3, caractérisé en ce qu'une partie des cations alcalins ou alcolino-terreux qui sont présents dans la clinoptilolite naturelle, sont remplacés par des ions d'hydrogène.
